# EUROPEAN PATENT APPLICATION

(11) **EP 1 985 290 A1**
(43) Date of publication of application: **29.10.2008**
(21) Application number: 07008507.1
(22) Date of filing: 26.04.2007
(51) Int. Cl.: A61K 9/70, A61L 15/58, A61F 13/02

(54) **Skin application medicament, method of applying the same to skin and method of manufacturing the same**

(71) Applicant: Sansho Cosme Inc., Sumiyosi-ku Osaka-shi Osaka (JP)
(72) Inventor: So, Yukio, Osaka-shi, Osaka (JP); Hatanaka, Katsuhito, Osaka-shi, Osaka (JP)
(74) Representative: Schildberg, Peter

(57) **Abstract**

The skin application medicament is used for treating or mitigating various symptoms on the skin, such as pimples, acne, comedo, winkles, rough skin, blotches, freckles, or the like.

Generally, there is hitherto employed an application sheet comprising a predetermined amount of medicinal substances with other substances applied on unwoven fabric or foamed plastics.

For example, there has been adopted an application sheet comprising a base agent called hydro-gel applied on unwoven fabric- However, the hydro-gel type application sheet is apt to dry and it is hard to keep the application sheet being stuck long.

The invention provides a skin application medicament comprising a base agent and a release sheet, the base agent made of mixture of: copolymer of styrene and diene compound; hydrocarbon; ester having carbons in number of 10 tc 25; and a medicinal constituent, the base agent being applied on the release sheet.

## Description

### BACKGROUND OF THE INVENTION

### Field of the Invention

The present invention relates to a skin application medicament, a method of applying the skin application medicament to skin, and a method of manufacturing the skin application medicament.

### Prior Art

The skin application medicament is used for treating or mitigating various symptoms on the skin, such as pimples, acne, comedo, wrinkles, rough skin, blotches, freckles, or the like.

Treating the skin is hitherto carried out mainly in the way of applying a medicinal ointment or cream which contains a medicinal constituent.

The liniment, i.e. the medicinal ointment or cream has such defects that the liniment does unexpectedly spread on the users' hands or to their clothes, or foreign objects such as dirt possibly stick to such users' hands or the clothes having the spread liniment. Furthermore, it is practically difficult to precisely apply such liniment in a predetermined or fixed quantity.

Therefore, there is hitherto employed an application sheet comprising a predetermined amount of medicinal substances with other substances applied on unwoven fabric or foamed plastics. In detail, such an application sheet is given previously a required amount of application of the medicinal and other substances. Hence, that the liniment is excessively applied to skin is avoided. Also, since the applied medicinal and other substances are covered with the unwoven fabric or foamed plastics, there is not the defect that the medicinal and other substances spread and stick to clothes and others, and foreign objects are caught there.

Some of this kind of application sheet have the medicinal substances which themselves have adhesive effect, and others of the application sheets have such adhesive effect possessed by the foregoing "other substances" than the medicinal ones. The latter such feature that the "other substances" than the medicinal ones have the adhesive effect is problematical in respect of necessitating enlargement of the whole of area. Thus, the mainstream one is the feature that the medicinal substances themselves have the adhesive effect.

For example, there has been adopted an application sheet comprising a base agent called hydro-gel applied on unwoven fabric. Hydro-gel is made of polyacrylic acid and polyacrylate having bridging therebetween. Hydro-gel contains therein a medicinal constituent and the medicinal constituent itself has the adhesive effect. However, the hydro-gel type application sheet is apt to dry and it is hard to keep the application sheet being stuck long.

Moreover, the application sheet having the base agent applied on the unwoven fabric or the like is not transparent and skin is not seen through the sheet. That is, changes and state of skin are not clear to users unless the unwoven fabric or the like is released. It is not easy to determine the effect of the medicinal substances and time to release the unwoven fabric or the like.

Also, it is a defect that presence of the unwoven fabric makes quite conspicuous skin being treated.

### SUMMARY OF THE INVENTION

Under the above circumstances, the inventor zealously continued to study and achieved the skin application medicament according to the present invention characterized in that a base agent comprises a mixture of: copolymer of styrene and diene compound; hydrocarbon; ester having carbons in number of 10 to 25; and a constituent having a medical effect, and the base agent is applied on a release sheet.

### BRIEF DESCRIPTION OF THE DRAWINGS

Fig. 1 is a sectional view showing an example of the skin application medicament according to the present invention.
Fig. 2 is a sectional view showing an exemplified use of the skin application medicament according to the present invention.
Fig- 3 is a sectional view showing another example of the skin application medicament according to the present invention.
Fig. 4 is a sectional view showing a state of use of the skin application medicament exemplified in Fig. 3.

### DETAILED DESCRIPTION OF THE INVENTION

The copolymer of styrene and a diene compound defined in the claims is a block copolymer of a diene compound (e.g. isoprene or butadiene) and styrene. And the remaining double bond may be (or not) subjected to hydrogenation to be saturated. In particular, hydrogenated styrene-butadiene block copolymer (SEBS) and hydrogenated styrene-isoprene block copolymer showed an excellent result. They are solid at ordinary or room temperature and not soluble in water but soluble in fats and oils.

Hydrocarbon for use for the present invention is generally in liquid state but may employ those in solid state at ordinary or room temperature, such as liquid paraffin, squalane, or vaseline, etc.

Particularly preferable and suitable is hydrocarbon in liquid state which is relatively low in viscosity (e.g. liquid paraffin). Viscosity, in particular, kinematic viscosity may be 40 to 350mm²/s. Liquid paraffin relatively low in viscosity is provided for dissolving the foregoing copolymer of styrene and the diene compound to thereby provide adhesion (stickiness) and efficiency of dissolving Medicinal substances.

Ester having carbons in number of 10 to 25 may be isopropyl palmitate (C₁₅H₂₁COOCH(CH₃)₂), isopropyl myristate (C₁₃H₂₇COOCH(CH₃)₂), etc, those being in liquid state at room or ordinary temperature, not soluble in water but soluble in fats and oils. A characteristic of the present invention is to mix the ester. The inventor found that to mix the ester enables the application sheet to be smoothly released from skin. Isopropyl myristate showed most preferable effect. Controlling loads of the ester enables stickiness to be quite readily adjusted.

The wording "medicinal" is directed to function itself of the skin application sheet and, particularly, the object aimed by applying the sheet, i.e., effects of skin whitening or lightening, anti-wrinkling, mitigating aging, anti-inflammatory, antimicrobial activity, a moisturizing effect, antioxidation, slimming, cleansing, and improving blood circulation.

The medicinal substances may employ those as follows.

Fats and oils may be avocado oil, camellia oil, corn oil, olive oil, sesame oil, castor cil, safflower oil, jojoba oil, liquid paraffin, squalane, paraffin, vaseline, micracrystalline wax, or the like. Moisturizing agents may be glycerin. 1,3-biatylene glycol, sorbitol, maltitol, hyaluronic acid, or the like. Blood circulation promoting agent may be capsaicin, caffeine, nicotin tocopherol, etc. There may be also listed up vitamin A, retinol, retinol palmitate, tetrahexyldecane ascorbinate, tocopherol, dl-α-tocopherol, dl-α -tocopherol acetate, L-menthol, camphor, sulfur, and, pyridoxine chloride.

Medicinal extracts usable for the present invention may be Phellodendoron amurense Ruprecht extract, licorice extract, Saxifraga extract, aloe extract, seaweed extract, ginseng extract, mulberry extract, etc.

What applicable to the present invention is any medicaments, such as substances effective for stiff shoulders or neck, an antifebrile, other poultice, and a heart medicine, employed in any features or treatment that medicament is to be applied to skin.

Those medicinal substances may be dissolved or not dissolved in the foregoing constituents of the base agent and therefore may employ water-soluble liquid or solid and oil-soluble liquid or solid.

Furthermore, an anti-oxidizing agent, such as BHT or the like may be mixed in the invention for preservation stability. BHT may be 3,5-ditertiarybutyl-4-hydroxytoluene. Also, scent, essential oil, etc, such as rose oil, peppermint oil, lavender oil, may be mixed to enable the invention to be usable in a manner of being perfume or to show an aromatherapy effect.

Mixing amounts (contents) of those constituents of the present invention may be, preferably, 25 to 60wt% (weight percent) of copolymer of styrene and diene compound, 40 to 70wt% of hydrocarbon, 0.5 to 15wt% of ester, and 0.5 to 24wt% of medicinal substances- In case that materials are in plural Zinds in the respective group of those constituents of the invention, the sum of mixing amounts of such materials should correspond to the above-mentioned values.

The release sheet may be a plastic sheet, plastic film or the like and preferably made of a material of synthetic resin such as PET (polyethylene terephthalate). The member in the form of sheet may be subjected to printing or coloring.

The release sheet is to be peeled and removed after the skin application medicament is applied to skin and is a kind of substrate. The release sheet may be transparent or colored but preferably to be transparent, enabling state of stickiness of the skin application medicament to skin to be well seen through before and upon removing the release sheet.

Thickness of application of the base agent should not be limited to any but may be about 0.05 to 1mm.

Such manner or method that the "substrate" is removed to leave only the base agent on skin is the large characteristic of the present invention. According to the present invention, an amount and thickness of application is able to be made constant of uniform in comparison with liniment filled in a bottle or tube.

The release sheet is peeled away and removed and the base agent is exposed. A surface agent may be provided in the invention for mitigation of viscosity on the surface of the exposed base agent. The surface agent may be preferably a release agent, such as silicon resin, fluorine resin, etc. The surface agent is applied between the release sheet and the base agent, so that the base agent has almost no stickiness on its surface and does not spread and not stick to clothes and others of users and also not catch dirt, etc.

It is preferable to provide a covering material (a film or a sheet) for preventing the base agent from being dirtied before applying the skin application medicament to skin. The covering material is to be peeled away and removed before applying the skin application medicament to skin.

The method of manufacturing the skin application medicament according to the present invention involves applying the foregoing base agent on the release sheet. An easy way of applying the base agent on the release sheet is carried out with viscosity being adjusted (by heating or the like). It is naturally not possible to stick the base agent to skin even at ordinary temperature if stickiness does not remain. Moreover, in case of employing the covering material, the base agent may be applied on the covering material, and the release sheet is stuck on the base agent.

In case of using the surface agent, the method of manufacturing as follows is useful.

First, the medicinal substances are applied on the covering material, the surface agent is then applied on the applied medicinal substances, and finally, the release sheet is stuck thereon.

Applying or sticking the skin application medicament to skin is carried out in such way that the covering material when provided is peeled away and removed and the skin application medicament is then stuck to skin. After leaving to stand for several dozens of seconds to about three minutes (Example 1), the release sheet is peeled away and removed. It completes through these steps.

### PREFERRED EMBODIMENTS OF THE INVENTION

Next, the present invention will be further detailed with referring to the examples.

### Example 1

The following constituents are mixed in a mixing apparatus.

| | | |
|---|---|---|
| 1 | SEES (hydrogenated serene-butadiene block copolymer, styrere content: about 35%) | 20.0wt% |
| 2 | Liquid paraffin | 71.5wt% |
| 3 | Isopropyl palmitate | 3.0wt% |
| 4 | Vitamin E | 5.0wt% |
| 5 | BHT | 0.5wt% |

If viscosity is high, it may be heated to 50 to 100°C.

The base agent is applied thinly (about 0.5mm) on the release sheet (plastic sheet) and a covering film is put on the base agent, achieving the skin application medicament. (Example 1 (Ex.1))

Table 1 shows also Examples 2 to 5 provided in the same manner with different mixing amounts. Values in the table are shown with weight percent. Also shown are Comparative Examples(C.E.) not containing styrene block copolymer, hydrocarbon, and ester.

| | | Ex.1 | Ex.2 | Ex.3 | Ex.4 | Ex.5 | C.E.1 | C.E.2 | C.E.3 |
|---|---|---|---|---|---|---|---|---|---|
| Constituents | SEBS | 20.0 | 30.0 | 40.0 | 30.0 | 30.0 | 30.0 | 91.5 | - |
| | Hydrocarbon | 71.5 | 61.5 | 51.5 | 46.5 | 44.5 | 62.5 | - | 91.5 |
| | IPP | 3.0 | 3.0 | 3.0 | 10.0 | 15.0 | - | 3.0 | 3.0 |
| | Vitamin E | 5.0 | 5.0 | 5.0 | 12.0 | 10.0 | 5.0 | 5.0 | 5.0 |
| | BHT | 0.5 | 0.5 | 0.5 | 0.5 | 0.5 | 0.5 | 0.5 | 0.5 |
| Stickiness | | Δ | ○ | ○ | ○ | ○ | Δ | × | × |
| Releassbility | | Δ | ○ | ○ | ○ | ○ | × | × | × |

| | | | | | | | | | |
|---|---|---|---|---|---|---|---|---|---|
| IPP:lsopropyl palmitate | | | | | | | | | |

It will be appreciated from Table 1 that when any one of styrene block copolymer, hydrocarbon and ester is missing, the effect of the present invention is now shown.

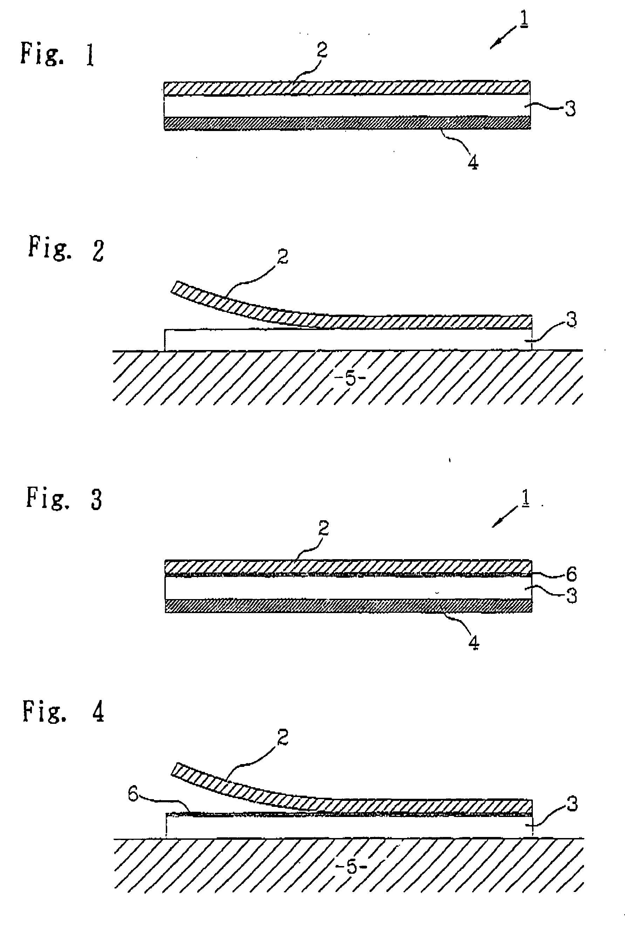

Fig. 1 is a sectional view showing an example of the skin application matter according to the present invention. The base agent 3 is applied on the release sheet 2, and a covering film 4 is stuck on the base agent 3. Usage of the invention is that the covering film 4 is peeled away and the skin application matter is stuck to skin. After a while of keeping left to stand, the release sheet 2 is peeled away, completing the operation.

Fig. 2 shows the skin application medicament 1 according to the present invention being stuck on skin 5 with the release sheet 2 being peeled.

Fig. 3 shows an example employing the surface agent 6 existing between the release sheet 2 and the base agent 3. The skin application matter is manufactured in such way that a medicinal substance or constituent 3 is applied on the covering material 4, the surface agent 6 is applied on the medicinal constituent, and finally, the release sheet 2 is stuck thereon.

Fig. 4 shows the example shown in Fig. 3 being stuck on skin 5 with the release sheet 2 being peeled. After peeling the release sheet 2, the surface agent 6 covers the base agent 3 and does not cause users to feel stickiness even when they touch with their hands.

### Effect of the Invention

The skin application medicament according to the present invention has the following advantages.
(1) since ester having carbons in number of 10 to 25 is mixed with styrene block copolymer and hydrocarbon, an appropriate stickiness is obtained. Thus, the skin application medicament is not unexpectedly peeled and not fall but is able to be peeled away relatively readily without damaging skin when the invention is to be peeled away and removed.
(2) The skin application medicament comprises only the base agent but not a carrier made for example of unwoven fabric or the like, to thereby be transparent, so that the state of skin is visually well seen through. Thus, any points where the skin application medicament is to he stuck are easily found or confirmed.
(3) Since the skin application medicament is transparent resolution of comedo, exudation of sebum, and size of foam are visually confirmed.
(4) Since the skin application medicament is transparent, it is not conspicuous. This is a significant matter for women even indoors, particularly, in private houses.
(5) Since the skin application medicament is transparent, it is able to he colored for any purposes.
(6) since the invention does not employ unwoven fabric or the like, what to be disposed or thrown away is less, leading to cost-down.
(7) since a carrier such as unwoven fabric is not used, users do not feel a feeling of non-affinity, or stiffness, en their skin.
(8) The invention does not need to be tightly enclosed before use as those which employing water gel.
(9) Since the invention is in viscous liquid state, it freely penetrates in pores and others and resolves.
(10) The skin application medicament comprises only the base agent but not unwoven fabric or the like, to thereby have a larger area for volatilization, so that volatile matter well evaporates, whereby enabling perfumes or the like to be less required.

## Claims

1. A skin application medicament comprising a base agent and a release sheet, the base agent made of a mixture of: copolymer of styrene and diene compound; hydrocarbon; ester having carbons in number of 10 to 25; and a medicinal constituent, the base agent being applied on the release sheet.

2. A skin application medicament as set forth in Claim 1, wherein a surface agent exists between the release sheet and the medicinal constituent.

3. A skin application medicament as set forth in Claim 1 or 2 wherein a covering material is provided at a side of the medicinal constituent opposite to the release sheet.

4. A skin application medicament as set forth in claim 1 through 3 wherein mixing amounts of the constituents are 25 to 60wt% of copolymer of styrene and diene compound, 40 to 70wt% of hydrocarbon, 0.5 to 15wt% of ester, and 0.5 to 20wt% of medicinal constituent.

5. A method of applying a skin application medicament to skin involving such steps that a skin application medicament set forth in Claim 1 is stuck on skin in such manner that the medicinal constituent sticks to skin, and after a predetermined time, the release sheet is peeled away to cause the medicinal constituent to remain on skin.

6. A method of manufacturing a skin application medicament involving such steps that a medicinal constituent is applied on a covering material, a surface agent is applied on the medicinal constituent, and finally, a release sheet is stuck thereon.
